# EUROPEAN PATENT SPECIFICATION

(11) **EP 3 261 553 B1**
(45) Date of publication and mention of the grant of the patent: **20.05.2020**
(21) Application number: 16705653.0
(22) Date of filing: 03.02.2016
(51) Int. Cl.: A61B 10/02, A61B 10/04

(54) **VARIABLE STIFFNESS ASPIRATION NEEDLE**
ASPIRATIONSNADEL MIT VARIABLER STEIFIGKEIT
AIGUILLE D'ASPIRATION À RIGIDITÉ VARIABLE

(30) Priority: 26.02.2015 US 201562121110 P
(43) Date of publication of application: 03.01.2018
(73) Proprietor: Boston Scientific Scimed, Inc., Maple Grove, MN 55311-1566 (US)
(72) Inventor: DAYTON, Peter L., Brookline, MA 02445 (US); HOLLYER, Matthew B., Williamstown, VT 05679 (US); SHEPARD, Douglas C., Mansfield, MA 02048 (US)
(74) Representative: Vossius & Partner Patentanwälte Rechtsanwälte mbB
(86) International application number: PCT/US2016/016400
(87) International publication number: WO 2016/137700

(56) References cited:
- EP-A1- 2 624 762
- US-A1- 2007 016 099
- US-A1- 2014 276 051

## Description

### Background

Needle biopsy procedures are common for the diagnosis and the staging of disease. For example, a fine needle aspiration needle may be advanced through a working channel of an endoscope to a target tissue site. Although fine needle aspiration is a highly sensitive and specific procedure, it is often difficult to acquire a suitable sample under certain clinical situations. The more cells or tissue that can be acquired, the greater the potential for a definitive diagnosis.

EP 2 624 762 A1 describes a bone marrow harvesting device which includes a flexible bone marrow harvesting needle that can bend during operation to prevent the needle tip from piercing the inner cortical wall of the target bone. The needle defines an aspiration channel that defines an intake end that is recessed to reduce the instances that the aspiration channel will be fouled by bone particles or other debris within the sample region.

US 2007/016099 A1 discloses a medical device and methods for collecting a tissue sample from a patient's body. The device includes a first tube defining a lumen and an aperture at a distal end of the first tube. A second tube extends within the lumen of the first tube and defines a lumen and slots, where at least some of the slots have sharp edges configured to shear tissue. The second tube is movable relative to the first tube such that the sharp edges cut a plurality of samples from tissue disposed within the aperture.

US 2014/276051 A1 discloses systems, methods, and devices for delivering ablation fluid to a location of interest within a body. Ablation fluid delivery systems and devices include flexible delivery needles within which a plurality of smaller flexible needles is housed. The smaller flexible needles are configured to articulate or bend with respect to the flexible delivery needle to increase an effective delivery area for the ablation fluid. The smaller flexible needles include apertures or other openings to facilitate delivery of the ablation fluid to the location of interest.

### Summary

The present disclosure is directed to a needle for collecting a tissue sample, comprising a needle body extending along a longitudinal axis from a proximal end to a distal end including a sharp tip for penetrating a target tissue, the needle body including a channel extending therethrough for collecting a tissue sample therein when the distal end is inserted into the target tissue, a distal portion of the needle body having a first stiffness and a proximal portion of the body having a second stiffness less than the first stiffness and a sleeve extending along a length of the needle body to cover the proximal portion.

According to the invention, the sleeve extends along an interior surface of the needle body so that the tissue sample is collected therewithin.

In an exemplary embodiment, the sleeve may be removable from the needle body to contain the tissue sample.

In an exemplary embodiment, the proximal portion may include a plurality of openings extending laterally through a wall thereof, the openings being sized and shaped to prevent a tissue sample collected within the channel from being passed therethrough.

In an exemplary embodiment, each of the plurality of openings may be one of circular, ovoid, slotted, rectangular and diamond-shaped.

In an exemplary embodiment, the plurality of openings may be laser cut into the elongated body.

In an exemplary embodiment, the proximal portion may include a spiral pattern cut therealong.

In an exemplary embodiment, the proximal portion may be formed of an elastic spring member affixed to a proximal end of the distal portion.

In an exemplary embodiment, the proximal portion may be formed of a braided, mesh structure.

The present disclosure is also directed to a needle for collecting a tissue sample, comprising a needle body extending from a proximal end to a distal end and including a channel extending therethrough, the distal end including a sharp tip so that the distal end is insertable into a target tissue to collect a tissue sample within the channel, a distal portion of the needle body having a first stiffness and a proximal portion of the needle body being configured as a spring so that the proximal portion has a second stiffness less than the first stiffness.

In an exemplary embodiment, the proximal portion may be cut in a spiral pattern to define the spring.

In an exemplary embodiment, the proximal portion may be formed of an elastic spring member affixed to a proximal end of the distal portion

In an exemplary embodiment, the needle may further comprise one of a sleeve extending along the proximal portion and a coating applied along the proximal portion.

In an exemplary embodiment, in an unbent configuration, adjacent coils of the spring may contact one another.

The present disclosure is also directed to a method, comprising cutting a pattern through at least a portion of a wall defining a proximal portion of a needle body so that the proximal portion of the needle body has a stiffness smaller than a stiffness of a distal portion of the needle body, the needle body extending from a proximal end to a distal end and including a channel extending therethrough, the distal end including a sharp tip and placing a sleeve along a length of the needle body.

The invention is defined by claim 1. Preferred embodiments are defined by the dependent claims. Further embodiments disclosed herein are for exemplary purpose only and do not form part of the invention.

### Brief Description

Fig. 1 shows a longitudinal side view of a needle according to a first exemplary embodiment of the present disclosure;
Fig. 2 shows a lateral cross-sectional view of the needle of Fig. 1, including a sleeve extending over an exterior surface of the needle;
Fig. 3 shows a lateral cross-sectional view of the needle of Fig. 1, including a sleeve extending along an interior surface of the needle;
Fig. 4 shows a longitudinal side view of a needle according to a second exemplary embodiment of the present disclosure;
Fig. 5 shows a longitudinal side view of a needle according to a third exemplary embodiment of the present disclosure;
Fig. 6 shows a longitudinal side view of a needle according to a fourth exemplary embodiment of the present disclosure; and
Fig. 7 shows a longitudinal side view of the needle of Fig. 6 with a coating extending over a proximal portion thereof.

### Detailed Description

The present disclosure may be further understood with reference to the following description and the appended drawings, wherein like elements are referred to with the same reference numerals. The present disclosure related to endoscopic devices and, in particular, devices for obtaining tissue samples. Exemplary embodiments of the present disclosure describe a needle including a distal portion having stiffness sufficient for facilitating insertion thereof into a target tissue and a proximal portion proximal the distal portion having sufficient flexibility to permit navigation of the needle through tortuous paths of a patient body. As will be described in greater detail below, the needle may be modified to increase a flexibility of the proximal portion via one of cutting, heat treating the steel and/or by removing material with a laser cutting machine. It should be noted that the terms "proximal" and "distal" as used herein, are intended to refer to a direction toward (proximal) and away from (distal) a user of the device.

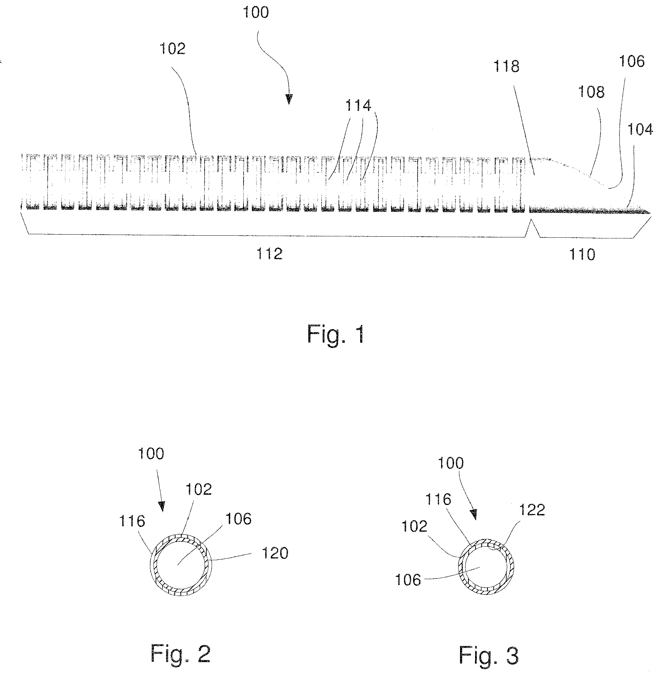

As shown in Figs. 1 - 3, a needle 100 according to a first exemplary embodiment of the present disclosure comprises an elongated needle body 102 extending along a longitudinal axis from a proximal end (not shown) to a distal end 104 and a channel 106 extending longitudinally therethrough. A distal portion 110 of the needle body 102 has a first bending stiffness sufficient to facilitate penetration of the distal end 104 into target tissue to collect a tissue sample within the channel 106. A proximal portion 112 of the needle body 102 proximal of the distal portion 110 includes a plurality of openings 114 extending laterally through a wall of the needle body 102 such that the proximal portion 112 has a second bending stiffness smaller than the bending stiffness of the distal portion 110 to increase a flexibility of the proximal portion 112 of the needle body 102 while maintaining the column strength necessary to enable the distal portion 110 to penetrate the target tissue. Thus, the needle 100 is stiff enough to efficiently acquire a good tissue sample within the channel 106 while also being flexible enough to navigate through even tortuous paths in a living body (e.g., along the path of a natural body lumen) to the target tissue site.

The distal end 104 of the needle body 102 includes a sharp tip 108 for piercing target tissue into which it is inserted and a cutting edge surrounding the sharp tip 108 to cut a tissue sample received within the channel 106 from surrounding tissue. The distal portion 110 of the needle body 102, including the sharp tip 108, extends from the distal tip 108 proximally along the needle body 102 from between 0.1 to 2.0 cm. The distal portion 110 of this embodiment is devoid of openings so that the distal portion 110 maintains the stiffness required for penetration of the sharp tip 108 into the target tissue.

The proximal portion 112 extends proximally from a proximal end 118 of the distal portion 110 and includes the plurality of openings 114 extending laterally through a wall defining the needle body to reduce a bending stiffness of the needle 100 along the proximal portion 112. For example, the proximal portion 112 may be approximately 50 to 90% as stiff as the distal portion 110, and more specifically, approximately 60% to 80% as stiff as the distal portion 110. A length of both the distal and proximal portions 110, 112 may vary. In one example, the proximal end 118 of the distal portion 110 may be immediately proximal to a proximal end of the sharp tip 108. In another example, the proximal end 118 of the distal portion may be distanced from the proximal end of the sharp tip 108. The proximal portion 112 including the openings 114 may extend along a length of the needle body 102 ranging from between 1.0 and 5.0 cm - the openings 114 are not required to extend to the proximal end of the needle body 102. A length of the proximal portion, however, may vary according to a length of the needle body 102.

The openings 114 may extend through an entire thickness of the wall so that the channel 106 is open to an exterior of the needle 100 via the openings 114. The openings 114 may extend through the proximal portion 112 to vary a stiffness along a length thereof. For example, a number of openings 114 per cm2 and/or size thereof may increase or decrease along the length of the proximal portion 112. The openings 114 may take any of a variety of shapes (e.g., circular holes, slots, diamond holes) so long as the openings 114 are sized to prevent the passage of tissue therethrough so that tissue collected within the channel 106 is retained therein. In an exemplary embodiment, the openings 114 may have a diameter ranging from between approximately 0.001 inches (5.61 x 10⁻⁶ cm2) 0.050 inches (1.29 x 10⁻² cm²). The openings 114 may be formed by, for example, laser cutting the needle body 102. The needle body 102 may be formed of a metal material such as, for example, stainless steel or nitinol, which is laser cut along the proximal portion 112 to form the openings 114. Alternatively, the needle body 102 may be formed of high modulus polymer materials used as metal replacements such as, for example, PEEK or LCPs, with or without reinforcement such as carbon fiber or glass.

Further, the needle body 102 may be coated with a low friction material or enveloped in a sleeve 116, as shown in Figs. 2 and 3, to protect the tissue sample or facilitate removal thereof. Low friction materials may include, for example, PTFE, other fluorinated polymers, parylene, or hydrophilic coatings. The coating/sleeve 116 may extend along an exterior surface 120 of the needle body 102, as shown in Fig. 2, and/or an interior surface 122 of the needle body 102, as shown in Fig. 3. Where the sleeve 116 extends along an interior of the needle body 102 the sleeve 116 may, optionally, be removable from the needle body 102 so that any tissue sample collected within the channel 106 may be packaged until analyzed. The tissue sample may be removed therefrom by cutting open the sleeve 116. In another embodiment, fluid pressure or a mandrel may be used to push the tissue sample out of the sleeve.

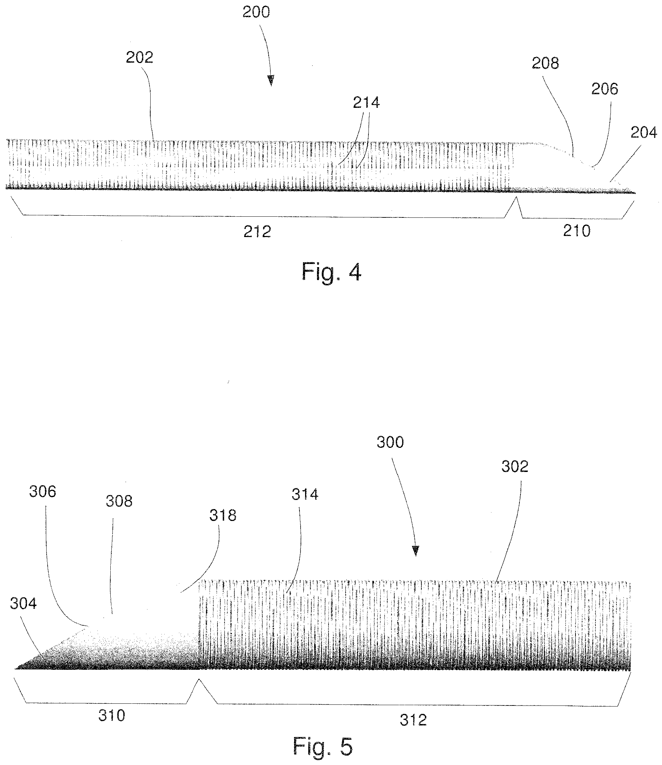

As shown in Fig. 4, a needle 200 according to a second exemplary embodiment of the present disclosure is, except as noted below, substantially similar to the needle 100, comprising an elongated needle body 202 extending along a longitudinal axis from a proximal end (not shown) to a distal end 204 and including a channel 206 extending longitudinally therethrough. The distal end 204 includes a sharp tip 208 with a cutting edge for penetration of target tissue so that a tissue sample may be collected within the channel 206. Similarly to the needle body 102, the needle body 202 includes a distal portion 210 having a first stiffness and a proximal portion 212 extending proximally therefrom and having a second stiffness smaller than the first stiffness permitting the needle 202 to be navigated through even tortuous paths of the body while maintaining a stiffness and column strength required to enable the distal portion 210 to be inserted into the target tissue. Rather than openings, however, the reduced stiffness of the proximal portion 212 is achieved via cutting a spiral (e.g., helical) pattern 214 along the proximal portion 212 of the needle body 212.

The spiral pattern 214 may be formed via a laser cutting the proximal portion helically along the length of the proximal portion 212. The spiral pattern 214 according to this embodiment extends through the entire wall thickness of the wall of the needle body 202. The spiral pattern 214 may extend along the entire length of the proximal section 210. Alternatively, the spiral pattern 214 may extend along portion(s) of the proximal section 219 at strategic points therealong which may be required to flex during use of the needle 200. The spiral pattern 214 defines the proximal portion 212 as a spring, which is particularly suited for providing both flexibility and column strength. The spiral pattern 214 is formed so that spacing between adjacent coils of the spiral pattern 214 during flexure is sufficiently small to prevent the tissue sample collected within the channel 206 from passing therethrough. For example, the space between adjacent coils of the spiral pattern may be no greater than 0.050 inches (0.127 cm). In an ideal configuration, when the needle 200 is in a straight (i.e., unbent) configuration, there would be no space between adjacent coils of the spiral pattern 214. In a bent or flexed configuration, any bending of the needle 200 would be outside (e.g., proximal) of a tissue acquisition portion of the needle 200 so that the tissue sample would be prevented from passing through any space between adjacent coils resulting from the flexure of the needle 200. In a further embodiment, a sleeve, substantially similar to the sleeve 116 described above with respect to the needle 100, may extend over an exterior of the proximal portion 212 and/or along an interior surface of the proximal portion 212 to prevent buckling under compression and to maintain the collected tissue sample within the channel 206.

As shown in Fig. 5, a needle 300 may be substantially similar to the needle 200 described above, comprising a needle body 302 extending along a longitudinal axis from a proximal end to a distal end 304 with a sharp tip 308 and including a channel 306 extending therethrough. Similarly to the needle body 202, the needle body 302 includes a distal portion 310 having a first stiffness and a proximal portion 312 having a second stiffness smaller than the first stiffness. Rather than cutting a spiral pattern into the proximal portion 312, however, the proximal portion may be formed via a spring element 314 coupled to a proximal end 318 of the distal portion 310. The spring element 314 according to this embodiment is formed of an elastic material wound about the longitudinal axis of the needle 300 in a substantially helical pattern. Similarly to the needle 200, in compression (as in during tissue acquisition), adjacent coils of the spring element 314 may contact one another so that no space exists therebetween. Thus, a tissue sample may be received within the channel 306 defined thereby. Any bending of the spring element 314 would be proximal of a tissue acquisition portion thereof so that the tissue sample is prevented from passing between adjacent coils thereof. In a further embodiment, the needle 300 also comprises a sleeve extending about the proximal portion 312 to prevent buckling of the needle body 302 under compression and/or prevent the tissue sample collected within the channel 306 from passing through the spaces between adjacent coils of the spring. The sleeve extending over the proximal portion 312 may be substantially similar to the sleeve 116 shown and described above in regard to the needle 100.

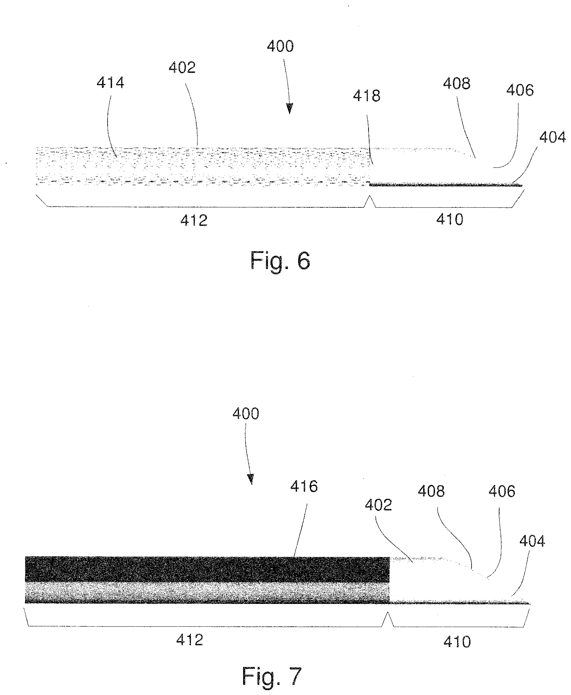

As shown in Figs. 6 - 7, a needle 400 may be substantially similar to the needle 100 - 300 described above, comprising a needle body 402 extending along a longitudinal axis from a proximal end to a distal end 404 with a sharp tip 408 and including a channel 406 extending therethrough. Similarly to the needle bodies 102 - 302 described above, the needle body 402 includes a distal portion 410 having a first stiffness sufficient to facilitate penetration of the distal end 402 into a target tissue and a proximal portion 412 having a second stiffness smaller than the first stiffness to increase a flexibility of the needle 400, permitting the needle to be navigated along tortuous paths in the body. The proximal portion 412 according to this embodiment is formed of a braided, mesh-like structure formed by cutting into a surface of a proximal portion 412 of the needle body 402 via, for example, laser cutting. In another embodiment, a separate cylindrical mesh structure is coupled to a proximal end 418 of the distal portion 412 via, for example, welding. The braided/mesh structure may then be coated with a polymer coating 416 to prevent the passage of a collected tissue sample within the channel 406 from passing therethrough.

According to an exemplary method of the present disclosure, a proximal portion of a needle comprising a needle body extending from a proximal end to a distal end and including a channel extending therethrough for collecting a tissue sample therein. The proximal portion is modified to decrease a stiffness of the proximal portion relative to the distal portion. The proximal portion of the needle may be modified according to any of the embodiments of the needles 100 - 400 described above. For example, the proximal portion may be cut (e.g., via laser cutting) with flexibility enhancing patterns such as the plurality of openings 114, described above with respect to the needle 100, a spiral pattern 214, as described above with respect to the needle 200 or a braided mesh pattern 414, as described above in regard to the needle 400. In another example, the proximal portion of the needle body may be modified by affixing to a distal portion of the needle body (including a sharp tip for piercing tissue) a flexible member such as a spring 314, as described above in regard to the needle 300, or a mesh structure 414, as described above in regard to the needle 400.

Any of the needles 100 - 400 may additionally be treated with a low-friction coating applied over the proximal portion. In another embodiment, a low-friction sleeve (e.g., sleeve 116, sleeve 416) may be applied over the proximal portion. Low-frictions materials such as PTFE, other fluorinated polymers, parylene, or hydrophilic coatings. The coating and/or sleeve may prevent buckling under compression, protection of the tissue sample collected within the channel of the needle body and/or facilitating retrieval of the tissue sample. The coating/sleeve would add little to the bending stiffness of the proximal portion over which it is applied.

In another embodiment, the proximal portion of the needle may be modified by heat treating the steel. The proximal portion of the needle body may be heated above a critical temperature and allowed to cool to, increasing an elasticity therealong. For example, where the needle is formed of Nitinol, the proximal portion of the needle may be heated to between approximately 400°C to 575°C, and more preferably between 475° C to 525° C. Where the needle is formed of stainless steel, the proximal portion may be heated to between approximately 205°C and 595°C.

It will be apparent to those skilled in the art that variations can be made in the structure and methodology of the present disclosure, without departing from the scope of the disclosure. Thus, it is intended that the present disclosure cover the modifications and variations of this disclosure provided that they come within the scope of the appended claims.

## Claims

1. A needle (100, 200, 300, 400) for collecting a tissue sample, comprising:
a needle body (102, 202, 302, 402) extending along a longitudinal axis from a proximal end to a distal end (104, 204, 304, 404) including a sharp tip (108, 208, 308, 408) for penetrating a tissue, the needle body including a channel (106, 206, 306, 406) extending therethrough for collecting the tissue sample therein when the distal end (104, 204, 304, 404) is inserted into the tissue, a distal portion (110, 210, 310, 410) of the needle body having a first stiffness and a proximal portion (112, 212, 312, 412) of the needle body having a second stiffness less than the first stiffness; and
a sleeve (116) extending along a length of the needle body to cover the proximal portion (112);
wherein a cutting edge surrounds the sharp tip (108, 208, 308, 408); and
wherein the sleeve (116) extends along an interior surface (122) of the needle body so that the tissue sample is collected therewithin.

2. The needle of claim 1, wherein the sleeve (116) is removable from the needle body (102, 202, 302, 402) to contain the tissue sample.

3. The needle of claim 1 or 2, wherein the proximal portion (112, 212, 312, 412) includes a plurality of openings (114, 214, 414) extending laterally through a wall thereof, the openings being sized and shaped to prevent a tissue sample collected within the channel from being passed therethrough.

4. The needle of claim 3, wherein each of the plurality of openings (114, 214, 414) are one of circular, ovoid, slotted, rectangular and diamond-shaped.

5. The needle of any one of claims 3 to 4, wherein the plurality of openings (114, 214, 414) are laser cut into the elongated body.

6. The needle of claim 1 or 2, wherein the proximal portion (212) includes a spiral pattern (214) cut therealong.

7. The needle of claim 1 or 2, wherein the proximal portion is formed of an elastic spring member affixed to a proximal end of the distal portion.

8. The needle of claim 1 or 2, wherein the proximal portion is formed of a braided, mesh structure.

9. The needle of any one of claims 1 to 8, further comprising one of a sleeve extending along the proximal portion and a coating applied along the proximal portion.

10. The needle of claim 6 or 7, wherein the proximal portion is configured as a spring and wherein, in an unbent configuration, adjacent coils of the spring contact one another.

## Patentansprüche

1. Nadel (100, 200, 300, 400) zur Entnahme einer Gewebeprobe, aufweisend:
einen Nadelkörper (102, 202, 302, 402), der sich entlang einer Längsachse von einem proximalen Ende zu einem distalen Ende (104, 204, 304, 404) erstreckt und eine scharfe Spitze (108, 208, 308, 408) zum Eindringen in ein Gewebe umfasst, wobei der Nadelkörper einen sich durch diesen erstreckenden Kanal (106, 206, 306, 406) zum Aufnehmen der Gewebeprobe darin umfasst, wenn das distale Ende (104, 204, 304, 404) in das Gewebe eingeführt wird, wobei ein distaler Abschnitt (110, 210, 310, 410) des Nadelkörpers eine erste Steifigkeit und ein proximaler Abschnitt (112, 212, 312, 412) des Nadelkörpers eine zweite Steifigkeit aufweist, die geringer als die erste Steifigkeit ist; und
sich eine Hülse (116) über eine Länge des Nadelkörpers erstreckt, um den proximalen Abschnitt (112) abzudecken;
wobei eine Schneidkante die scharfe Spitze (108, 208, 308, 408) umgibt; und
wobei sich die Hülse (116) entlang einer Innenseite (122) des Nadelkörpers erstreckt, so dass die Gewebeprobe darin aufgenommen ist.

2. Nadel nach Anspruch 1, wobei die Hülse (116) aus dem Nadelkörper (102, 202, 302, 402) entfernt werden kann, um die Gewebeprobe aufzunehmen.

3. Nadel nach Anspruch 1 oder 2, wobei der proximale Abschnitt (112, 212, 312, 412) mehrere Öffnungen (114, 214, 414) umfasst, die seitwärts durch eine Wand von diesem verlaufen, wobei die Öffnungen so bemessen und geformt sind, dass eine im Kanal aufgenommene Gewebeprobe nicht durch sie passieren kann.

4. Nadel nach Anspruch 3, wobei jede der mehreren Öffnungen (114, 214, 414) eine der folgenden Formen aufweist: rund, eiförmig, schlitzförmig, rechteckig oder rhombenförmig.

5. Nadel nach einem der Ansprüche 3 bis 4, wobei die mehreren Öffnungen (114, 214, 414) mit Laser in den langgestreckten Körper geschnitten sind.

6. Nadel nach Anspruch 1 oder 2, wobei der proximale Abschnitt (212) ein entlang diesen geschnittenes Spiralmuster (214) umfasst.

7. Nadel nach Anspruch 1 oder 2, wobei der proximale Abschnitt aus einem elastischen Federelement gebildet ist, das an einem proximalen Ende des distalen Abschnitts angebracht ist.

8. Nadel nach Anspruch 1 oder 2, wobei der proximale Abschnitt aus einer geflochtenen Maschenstruktur gebildet ist.

9. Nadel nach einem der Ansprüche 1 bis 8, die ferner eine Hülse, die sich entlang des proximalen Abschnitts erstreckt, oder eine entlang des proximalen Abschnitts aufgebrachte Beschichtung aufweist.

10. Nadel nach Anspruch 6 oder 7, wobei der proximale Abschnitt als Feder ausgebildet ist und wobei benachbarte Windungen der Feder in einer nicht gebogenen Konfiguration miteinander in Kontakt stehen.

## Revendications

1. Aiguille (100, 200, 300, 400) pour collecter un échantillon de tissu, comprenant :
un corps d'aiguille (102, 202, 302, 402) s'étendant le long d'un axe longitudinal, d'une extrémité proximale à une extrémité distale (104, 204, 304, 404) comprenant une pointe pointue (108, 208, 308, 408) pour pénétrer un tissu, le corps d'aiguille comprenant un canal (106, 206, 306, 406) s'étendant à travers ce dernier pour y collecter l'échantillon de tissu, lorsque l'extrémité distale (104, 204, 304, 404) est insérée dans le tissu, une partie distale (110, 210, 310, 410) du corps d'aiguille ayant une première rigidité et une partie proximale (112, 212, 312, 412) du corps d'aiguille ayant une seconde rigidité inférieure à la première rigidité ;
un manchon (116) s'étendant le long d'une longueur du corps d'aiguille afin de recouvrir la partie proximale (112) ;
dans laquelle un bord de coupe entoure la pointe pointue (108, 208, 308, 408) ; et
dans laquelle le manchon (116) s'étend le long d'une surface intérieure (122) du corps d'aiguille de sorte que l'échantillon de tissu est collecté à l'intérieur de ce dernier.

2. Aiguille selon la revendication 1, dans laquelle le manchon (116) peut être retiré du corps d'aiguille (102, 202, 302, 402) pour contenir l'échantillon de tissu.

3. Aiguille selon la revendication 1 ou 2, dans laquelle la partie proximale (112, 212, 312, 412) comprend une pluralité d'ouvertures (114, 214, 414) s'étendant latéralement à travers sa paroi, les ouvertures étant dimensionnées et formées pour empêcher un échantillon de tissu collecté dans le canal, de passer à travers ces dernières.

4. Aiguille selon la revendication 3, dans laquelle chacune de la pluralité d'ouvertures (114, 214, 414) a l'une parmi une forme circulaire, une forme ovoïde, une forme fendue, une forme rectangulaire et une forme de diamant.

5. Aiguille selon l'une quelconque des revendications 3 à 4, dans laquelle la pluralité d'ouvertures (114, 214, 414) sont découpées au laser dans le corps allongé.

6. Aiguille selon la revendication 1 ou 2, dans laquelle la partie proximale (212) comprend un motif en spirale (214) découpé le long de cette dernière.

7. Aiguille selon la revendication 1 ou 2, dans laquelle la partie proximale est formée avec un élément de ressort élastique fixé sur une extrémité proximale de la partie distale.

8. Aiguille selon la revendication 1 ou 2, dans laquelle la partie proximale est formée avec une structure en maille tressée.

9. Aiguille selon l'une quelconque des revendications 1 à 8, comprenant en outre l'un parmi un manchon s'étendant le long de la partie proximale et un revêtement appliqué le long de la partie proximale.

10. Aiguille selon la revendication 6 ou 7, dans laquelle la partie proximale est configurée comme un ressort et dans laquelle, dans une configuration non pliée, des spires adjacentes du ressort sont en contact entre elles.
